# EUROPEAN PATENT SPECIFICATION

(11) **EP 0 909 565 B1**
(45) Date of publication and mention of the grant of the patent: **02.01.2004**
(21) Application number: 98119481.4
(22) Date of filing: 15.10.1998
(51) Int. Cl.: A61M 1/36, B01J 20/26

(54) **Use of a non-ionic adsorbent resin combined with the system known as bioartificial liver**
Verwendung eines nichtionischen Adsorptionsharzes in Kombination mit dem System einer bioartifiziellen Leber
Utilisation d'un résine adsorbante non-ionique combiné avec un système de foie bioartificiel

(30) Priority: 16.10.1997 IT MO970044 U
(43) Date of publication of application: 21.04.1999
(73) Proprietor: B. BRAUN CAREX S.p.A., 41037 Mirandola (Modena) (IT)
(72) Inventor: Gavioli, Giuliana, 41037 Mirandola, Modena (IT)
(74) Representative: Modiano, Guido, Dr.-Ing.

(56) References cited:
- US-A- 4 198 396
- PATENT ABSTRACTS OF JAPAN vol. 14, no. 109, 28 February 1990 & JP 01 313479 A (MATSUDAIRA TENNENBUTSU KENKYUSHO K.K.), 18 December 1989,
- PATENT ABSTRACTS OF JAPAN vol. 13, no. 158, 17 April 1989 & JP 63 315145 A (TERUMO CORP.), 22 December 1988,
- DATABASE WPI Week 8112 Derwent Publications Ltd., London, GB; AN 81-20221D XP002088063 & JP 56 008 059 A (TEIJIN LTD) , 27 January 1981

## Description

The present invention relates to the use of a non-ionic adsorbent resin in the extracorporeal absorption of bilirubin and bile acids.

In particular, the present invention relates to the use of a styrene-divinylbenzene copolymer resin having a macroreticular structure for absorbing bile acids and bilirubin in a bioartificial liver system.

Acute or fulminating hepatitis is a disease that affects the hepatic system with a swift clinical course and is mostly caused by infections produced by the hepatitis A, B, C virus, food toxinfections, drugs or other viral agents such as the cytomegalovirus or the Epstein-Barr virus.

Fulminating hepatitis requires, as basic therapy, a specific diet combined with a specific drug treatment. When the hepatic function is more severely compromised, hemodialysis and hemoperfusion are used in combination with bioartificial organs or systems.

Among these systems, the best-known one is constituted by the so-called bioartificial lever, a system which uses cultured hepatocytes with effective and constant metabolism and protein synthesis, capable of providing both detoxifying activity and synthesis activity.

It is known from scientific literature and from experimental studies that the bioartificial liver, based on the activity of hepatocytes, can provide both synthesis activity and detoxifying activity.

In particular, it has long been noted that isolated pig hepatocytes, cultured in a hollow-fiber bioreactor, metabolize bile acids during the perfusion of said bioreactor with human plasma enriched with bile acids.

During the execution of procedures for using bioartificial liver systems and particularly hollow-fiber bioreactors it has been observed that the hepatocytes, in performing the conjugation of cholic acids, accumulate cholates and taurocholates from the recirculating plasma.

Most of the cholate conjugation is already evident after three hours and the proportion of conjugated bile acids is always higher in the hepatocyte compartment (extrafiber space) than in the recirculating plasma (intrafiber space).

This phenomenon is a severe limitation to the use of bioreactors, since it leads to a very quick saturation of the artificial liver system based on bioreactors. This operating drawback is probably caused by the loss of the bile excretory system, which in vivo allows to eliminate metabolic products.

In order to obviate this severe drawback, two operating methods have been identified which are suitable to remove the excess bilirubin and bile acids in bioreactors based on hollow fibers:
-- the first method consists in creating a loop which allows to convey to the patient the plasma water collected in the extrafiber space, which is rich in bilirubin and bile acids. Accordingly, the accumulation of metabolites in the space dedicated to the culture of the hepatocytes (extrafiber space) is prevented (ELAD, Sussmann et al. in Hepatology, 1996);
-- the second method combines the bioartificial liver (BAL) system with an activated-charcoal filter which detoxifies in a nonselective manner the plasma intended for the cell cultures.

Both systems are currently used in the bioartificial liver system with the goal of removing the bilirubin and bile acids that arrive from said system or from the patient.

These operating methods, however, entail implementation drawbacks which make them scarcely efficient and scarcely suitable to achieve the intended goals.

The end result is that a high concentration of bilirubin and bile acids is recirculated if the bioartificial lever (BAL) system is not replaced frequently.

These drawbacks are mainly due to the modest selectivity of currently used systems for filtering out cholanic acids.

Activated charcoal in fact has modest selectivity in the absorption of cholanic acids and derivatives (bilirubin, bile acids).

It has also been observed that the system providing a loop in ELAD conveys the bile acids and derivatives to the blood collection pouches or to the patient, since it removes them only from the cell cultures of the artificial bioreactor.

The general aim of the present invention is therefore to avoid or substantially reduce the drawbacks of the prior art.

A general object of the present invention is to provide new uses for specific copolymer resins in the field of extracorporeal treatments of human blood or plasma from patients with compromised hepatic function.

A particular object of the present invention is to provide a device which allows, in the field of use of the bioartificial liver system, an effective removal of bile acids and bilirubin from the hepatic cell culture, preventing them from being subsequently infused to the patient.

In accordance with a first aspect of the present invention, a styrene-divinylbenzene copolymer resin is used for the manufacture of a biomedical device for the extracorporeal absorption of bilirubin and bile acids and derivatives thereof from human blood or plasma.

Within the scope of the present invention, the copolymer used preferably has a particle size between 300 and 1000 microns.

A resin that is particularly suitable to achieve the intended aim and objects of the invention has a macroreticular structure.

The resin used in the present invention is the result of an accurate selection performed with the goal of obtaining a product which is capable of performing a selective removal of bilirubin, bile acids and cholic acid derivatives from extracorporeal human plasma or blood without interfering with other blood components.

The selected resin is advantageously a non-ionic (chargeless) absorbent which has a specific attraction for nonpolar fat-soluble organic molecules. Said resin furthermore does not have an intrinsic pharmacological activity.

Preferably, said resin is an absorbent polymeric compound in the form of insoluble white pearls. Each pearl of resin is advantageously melted together with others as an agglomerate of cross-linked polystyrene microspheres. The resulting reticular structure provides a solid continuous phase to a continuous porous phase, so that water or other solvents easily penetrate through the pores. Each resin bed therefore has a very large surface area. A preferred surface area is between 650 and 850 m²/g, the value of 750 m²/g being the most preferred. The average diameter of the pores is preferably between 40 and 60 A^{o}, 50 A^{o} being the most preferred.

The highly aromatic structure of these materials gives them high affinity to the hydrophobic portions of organic molecules, making them more effective and specific than vegetable charcoal in absorbing organic solutes having hydrophobic and hydrophilic portions from aqueous solutions.

Said copolymer resin of the above-described type can be used to remove bilirubin and bile acids and derivatives from the extrafiber space of an artificial liver system based on hollow-fiber bioreactors in which said resin is integrated in the bioreactor or contained in a separate filter.

The use of said resins in combination with a bioartificial liver, for example of the type constituted by a bioreactor with hepatocytes, produces a synergistic detoxifying effect which is particularly important in the field of extracorporeal treatments of fresh or stored blood. Selective removal of bilirubin and bile acids is thus provided, preventing their accumulation in the cell culture (hepatocytes) and preventing them from being subsequently reinfused to the patient and also preventing rapid saturation of the bioreactor.

By way of example, the above-described copolymer resin can be used in combination with a bioreactor of the type disclosed in the copending European patent application in the name of this same Applicant, which claims the priority of Italian patent application no. MO97A000182.

According to another aspect, said copolymer resin can be used for the extracorporeal removal of bilirubin and bile acids and derivatives thereof from blood or plasma in an artificial liver system based on a hollow-fiber bioreactor in which said resin is integrated in the bioreactor or contained in a separate filter.

According to another embodiment of the present invention, there is provided a biomedical device for the extracorporeal absorption of bilirubin and bile acids and derivatives thereof from human blood or plasma according to claim 8.

Said biomedical device is constituted by a cartridge or capsule inside which the styrene-divinylbenzene copolymer is preferably arranged like a lattice, through the polymer meshes of which the blood or plasma can flow freely. The capsule is a hollow device which contains a sterile and nonpyrogenic isotonic saline solution and has an inlet and an outlet for the passage of the blood stream. Advantageously, the capsule is further provided with one or more filters which have, for example, a pore size of approximately 75 microns.

Further characteristics and advantages of the present invention will become apparent from the description of the following embodiments, illustrated only by way of non-limitative example with the aid of the accompanying drawings, wherein:
Figure 1 is a schematic view of the extracorporeal use of the resin according to the invention on human plasma;
Figure 2 is a schematic view of the extracorporeal use of the resin according to the invention in association with a bioartificial liver;
Figure 3 is a schematic view of the use of a cartridge which contains the resin according to the invention;
Figure 4 is a schematic view of an extracorporeal treatment which combines hemoperfusion and dialysis.

In all the figures, identical reference numerals designate identical elements.

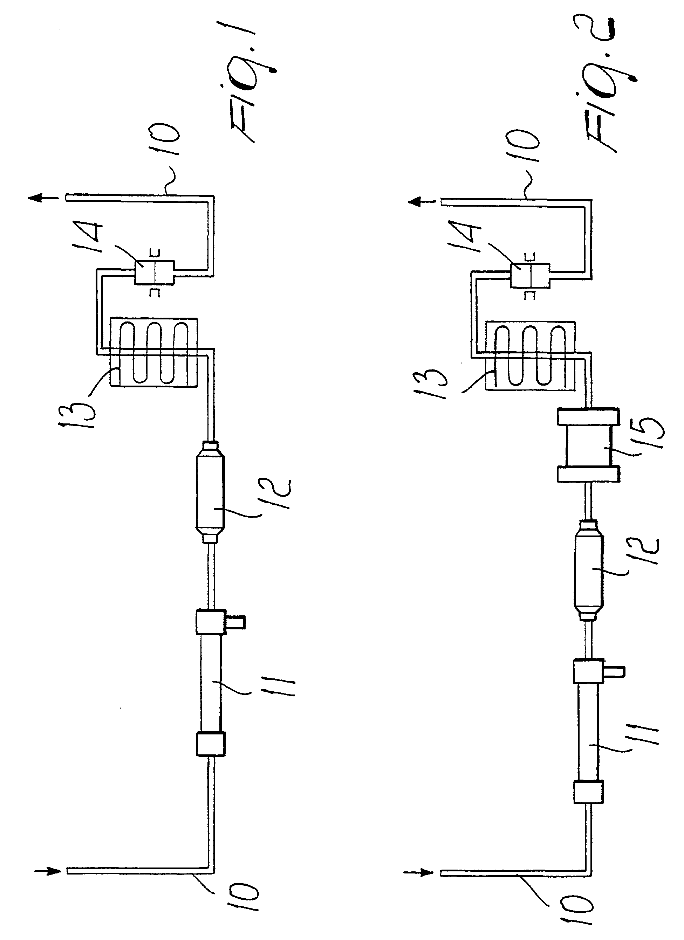

Figure 1 relates to an embodiment of the present invention and illustrates an extracorporeal circuit 10 in which a stream of human blood, taken from collection pouches (not shown), flows. The blood stream is conveyed to a plasma separator 11, in which the corpuscle fraction is separated from the liquid one (plasma), which is sent to a cartridge 12 containing a polystyrene-divinylbenzene copolymer resin which is suitable to absorb the bile acids and bilirubin that it contains. At the outlet of the cartridge 12, the plasma stream passes through a heater 13 which brings the plasma to body temperature conditions (approximately 37.2°C). The plasma is then sent to a collection container 14, where it can be stored until it is to be used.

The flow of the blood through the extracorporeal circuit is advantageously maintained at a constant speed by using a conventional feed pump, which is not shown.

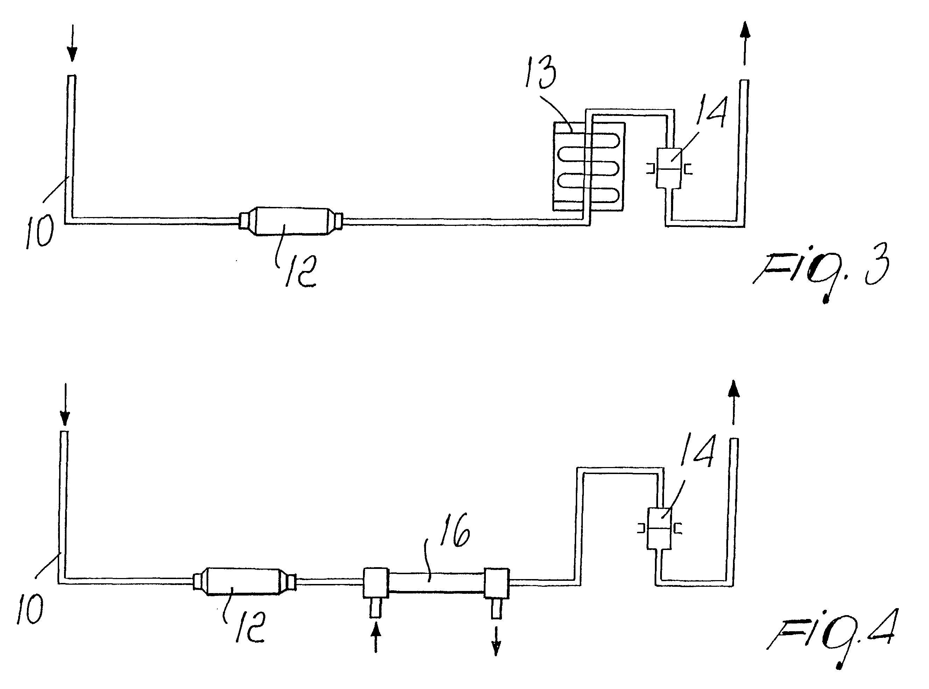

Figure 2 is a view of a second embodiment of the invention and illustrates an extracorporeal treatment circuit 10 which is supplied with human blood arriving from patients with altered blood parameters due to fulminating hepatitis. In particular, the plasma treatment along the extracorporeal circuit 10 provides for the combined use of a copolymer resin according to the invention and of an artificial liver system.

The blood stream is initially subjected to a treatment for separating the corpuscle fraction from the liquid fraction in a plasma separator 11. The plasma fraction is then subjected to a treatment for the adsorption of bile acids and bilirubin by using a polystyrene-divinylbenzene copolymer resin provided inside a cartridge 12. At the outlet of the cartridge 12, the plasma has a distinct reduction in the values of bilirubin and bile acids and is therefore in a form which is advantageously suitable for subjecting it to a detoxifying treatment inside a bioreactor with hepatocytes 15 (bioartificial liver). At the outlet of the bioreactor, the plasma is heated and then transferred to a suitable container 14, from which it can be infused to the patient or stored for subsequent infusions.

Figure 3 illustrates a third embodiment of the invention, in which an extracorporeal stream of blood arriving from a collection pouch (not shown) is made to pass within a cartridge 12 which comprises a polystyrene-divinylbenzene copolymer resin having a macroreticular structure. The blood at the inlet of the cartridge has high bilirubin values. During transit through the copolymer framework, the excess bilirubin and cholanic acid derivatives in the blood are absorbed so that the parameters of the blood at the outlet are within physiological limits.

The blood is then subjected to heating by irradiation in a heater 13 which has an electrically heated coil. At the end of the treatment, the blood is conveyed into a container 14 which is suitable for perfusion.

Figure 4 illustrates a combined hemoperfusion and dialysis treatment which provides for a step for treating the blood inside a cartridge 12 which contains a copolymer resin having a reticular structure according to the invention. The blood that leaves the cartridge is perfused in a dialyzer 16 of a conventional type. At the end of the treatment, the blood is transferred into a sterile container 14, where it is stored until it is to be used.

Experiments have shown the ability of the above-described copolymer resins to reduce the hematic value of bile acids by more than 90% after 20 minutes of treatment and to remove total bilirubin by more than 50%, again after 20 minutes.

Where technical fatures mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. Use of a styrene-divinylbenzene copolymer resin for the manufacture of the biomedical device according to claim 8 for the extracorporeal removal of bilirubin and bile acids and derivatives thereof from human blood and plasma.

2. The use according to claim 1, wherein said resin has a macroreticular structure.

3. The use according to claim 1, wherein the biomedical device is selected from the group consisting of cartridge and capsule.

4. The use according to any of claims 1 or 2, wherein said copolymer resin has a particle size between 300 and 1000 microns.

5. The use according to claim 1, wherein said copolymer resin has an average pore diameter from 40 to 60 Å.

6. The use according to claim 1, wherein said resin is integrated in a hollow-fiber bioreactor or contained in a separate filter.

7. The use according to any of claims 1, 2 or 4 wherein the biomedical device is an artificial liver system based on a hollow-fiber bioreactor and wherein said resin is integrated in the bioreactor or contained in a separate filter.

8. Biomedical device for the extracorporeal removal of bilirubin and bile acids and derivatives thereof from blood or plasma, the device comprising:
a) an element (12) selected from the group consisting of a cartridge and a capsule, which element has an inlet for the blood and an outlet for the plasma, and
b) a copolymer resin arranged within said element,
**characterised in that** said copolymer resin is a styrene-divinylbenzene copolymer resin.

9. The device according to claim 8, wherein the resin has a macroreticular structure.

10. Extracorporeal circuit (10) for the removal of bilirubin, bile acids and derivatives thereof from blood or plasma, the circuit comprising:
a) a plasma separator (11) for separating the blood flowing through said separator into plasma and blood cells, the plasma separator having an inlet for the blood and an outlet for the plasma, and
b) a biomedical device (12) according to claim 8 connected with the outlet of the plasma separator.

11. The extracorporeal circuit according to claim 10, further comprising a bioreactor (15) containing hepatocytes and operatively connected with the outlet for plasma of the biomedical device.

## Patentansprüche

1. Verwendung eines Styrol-Divinylbenzol-Copolymer-Harzes zur Herstellung der biomedizinischen Vorrichtung nach Anspruch 8 zur extrakorporalen Entfernung von Bilirubin und Gallensäuren sowie Derivaten davon aus menschlichem Blut und Plasma.

2. Verwendung nach Anspruch 1, wobei das Harz eine makroretikuläre Struktur aufweist.

3. Verwendung nach Anspruch 1, wobei die biomedizinische Vorrichtung aus der Gruppe bestehend aus Kartusche und Kapsel ausgewählt ist.

4. Verwendung nach Anspruch 1 oder 2, wobei das Copolymer-Harz eine Teilchengröße zwischen 300 und 1000 Mikrometer aufweist.

5. Verwendung nach Anspruch 1, wobei das Copolymer-Harz einen mittleren Porendurchmesser von 40 bis 60 Å aufweist.

6. Verwendung nach Anspruch 1, wobei das Harz in einen Hohlfaser-Bioreaktor integriert oder in einem separaten Filter enthalten ist.

7. Verwendung nach Anspruch 1, 2 oder 4, wobei die biomedizinische Vorrichtung ein künstliches Lebersystem auf der Basis eines Hohlfaser-Bioreaktors ist und das Harz in den Bioreaktor integriert oder in einem separaten Filter enthalten ist.

8. Biomedizinische Vorrichtung zur extrakorporalen Entfernung von Bilirubin und Gallensäuren sowie Derivaten davon aus Blut oder Plasma, die Folgendes umfasst:
a) ein Element (12), ausgewählt aus der Gruppe bestehend aus einer Kartusche und einer Kapsel, das eine Eintrittsöffnung für das Blut und eine Austrittsöffnung für das Plasma aufweist, und
b) ein in dem Element angeordnetes Copolymer-Harz,
**dadurch gekennzeichnet, dass** das Copolymer-Harz ein Styrol-Divinylbenzol-Copolymer-Harz ist.

9. Vorrichtung nach Anspruch 8, bei der das Harz eine makroretikuläre Struktur besitzt.

10. Extrakorporaler Kreislauf (10) für die Entfernung von Bilirubin, Gallensäuren und Derivaten davon aus Blut oder Plasma, der Folgendes umfasst:
a) einen Plasmaseparator (11) zum Auftrennen des durch den Separator fließenden Blutes in Plasma und Blutzellen, wobei der Plasmaseparator eine Eintrittsöffnung für das Blut und eine Austrittsöffnung für das Plasma aufweist, und
b) eine biomedizinische Vorrichtung (12) nach Anspruch 8, die mit der Austrittsöffnung des Plasmaseparators verbunden ist.

11. Extrakorporaler Kreislauf nach Anspruch 10, der weiterhin einen Leberzellen enthaltenden Bioreaktor (15) umfasst und mit der Plasmaaustrittsöffnung der biomedizinischen Vorrichtung betriebsbereit verbunden ist.

## Revendications

1. Utilisation d'une résine de copolymère styrène-divinylbenzène pour la fabrication du dispositif biomédical selon la revendication 8 pour l'élimination extracorporel de la bilirubine et des acides biliaires et de leurs dérivés du sang et du plasma humains.

2. Utilisation selon la revendication 1, dans laquelle ladite résine a une structure macroréticulaire.

3. Utilisation selon la revendication 1, dans laquelle le dispositif biomédical est choisi dans le groupe consistant en une cartouche et une capsule.

4. Utilisation selon l'une quelconque des revendications 1 ou 2, dans laquelle ladite résine de copolymère a une taille de particule entre 300 et 1000 microns.

5. Utilisation selon la revendication 1, dans laquelle ladite résine de copolymère a un diamètre moyen de pore de 40 à 60 Å.

6. Utilisation selon la revendication 1, dans laquelle ladite résine est intégrée dans un bioréacteur à fibres creuses ou contenue dans un filtre séparé.

7. Utilisation selon l'une des revendications 1, 2 ou 4, dans laquelle le dispositif biomédical est un système de foie artificiel basé sur un bioréacteur à fibres creuses, et dans laquelle ladite résine est intégrée dans le bioréacteur ou contenue dans un filtre séparé.

8. Dispositif biomédical pour l'élimination extracorporel de la bilirubine et des acides biliaires et de leurs dérivés du sang ou du plasma, le dispositif comprenant :
a) un élément (12) choisi dans le groupe consistant en une cartouche et une capsule, lequel élément a une entrée pour le sang et une sortie pour le plasma, et
b) une résine de copolymère disposée dans ledit élément,
**caractérisé en ce que** ladite résine de copolymère est une résine de copolymère styrène-divinylbenzène.

9. Dispositif selon la revendication 8, dans lequel la résine a une structure macroréticulaire.

10. Circuit extracorporel (10) pour l'élimination de la bilirubine, des acides biliaires et de leurs dérivés du sang ou du plasma, le circuit comprenant :
a) un séparateur de plasma (11) pour séparer le sang s'écoulant par ledit séparateur en plasma et cellules sanguines, le séparateur de plasma ayant une entrée pour le sang et une sortie pour le plasma, et
b) un dispositif biomédical (12) selon la revendication 8, connecté à la sortie du séparateur de plasma.

11. Circuit extracorporel selon la revendication 10, comprenant en outre un bioréacteur (15) contenant des hépatocytes, et connecté de manière opératoire à la sortie pour du plasma du dispositif biomédical.
